# EUROPÄISCHE PATENTANMELDUNG

(11) **EP 3 208 292 A1**
(43) Veröffentlichungstag der Anmeldung: **23.08.2017**
(21) Anmeldenummer: 17155989.1
(22) Anmeldetag: 14.02.2017
(51) Int. Cl.: C08G 59/14, C08G 59/50, C08G 59/68

(54) **MODIFIZIERUNGSMITTEL FÜR HÄRTBARE ZUSAMMENSETZUNGEN ENTHALTEND BENZYLALKOHOL-ALKOXYLATE**

(30) Priorität: 19.02.2016 EP 16156475
(71) Anmelder: Evonik Degussa GmbH, 45128 Essen (DE)
(72) Erfinder: Roland, Katrin, 45257 Essen (DE); Lobert, Matthias, 45309 Essen (DE); Kirchner, Jürgen, 45134 Essen (DE); Reuter, Ellen, 44799 Bochum (DE)

(57) **Zusammenfassung**

Die vorliegende Erfindung betrifft ein Modifizierungsmittel enthaltend Benzylalkoholbasierte Alkoxylate für härtbare Zusammensetzungen mit mindestens einem Epoxidharz und einem Härter, welche durch Umsetzung von Benzylalkohol mit Alkylenoxiden erhalten werden, wobei die Benzylalkohol-basierten Alkoxylate mindestens ein Ethoxy-Fragment aufweisen.

## Beschreibung

Die vorliegende Erfindung betrifft ein Modifizierungsmittel enthaltend Benzylalkohol-basierte Alkoxylate für härtbare Zusammensetzungen mit mindestens einem Epoxidharz und einem Härter, dessen Verwendung sowie härtbare Zusammensetzungen enthaltend besagtes Modifizierungsmittel.

Epoxidharze sind bekannte Rohstoffe für die Herstellung von qualitativ hochwertigen Gießharzen und Beschichtungsmassen. Die Reaktion dieser Harze mit einer Reihe von Härtern, insbesondere mit aminischen Härtern, führt zu vernetzten Polymeren, die duroplastisch sein können und in den Bereichen z. B. Civil Engineering (Bauwesen), besonders in Industrieböden, Abdichtungen und Betonsanierungsprodukten, Composites (Faserverbundwerkstoffen), Vergussmassen, Lacken und Klebstoffen, Verwendung finden können. Ein Überblick über die Harze und Härter sowie deren Verwendung im Bereich Civil Engineering einschließlich ihrer Eigenschaften findet sich in H. Schuhmann, "Handbuch Betonschutz durch Beschichtungen", Expert Verlag 1992, Seite 396 - 428. Der Einsatz der Harze und Härter für den Bereich Composites wird in P.K.Mallick, "Fiber-Reinforced Composites, Materials, Manufacturing, and Design", CRC Press, Seite 60 - 76 beschrieben.

Neben Epoxidharzen und Härtern enthält eine übliche härtbare Zusammensetzung auch Reaktivverdünner und nichtreaktive Bestandteile, wie zum Beispiel Katalysatoren, Additive, Weichmacher, Verschnitt- oder Modifizierungsmittel. Diese Bestandteile beeinflussen einerseits positiv die Reaktivität und den Verlauf der Zusammensetzung, andererseits kann die Umgebung durch kontinuierliche Ausdunstung der nach Abschluss der Härtung nicht kovalent eingebundenen nichtreaktiven Bestandteile belastet werden.

Ein langjährig etabliertes Modifizierungsmittel stellt Benzylalkohol dar. Benzylalkohol ist sowohl mit dem Epoxidharz als auch mit dem Aminhärter mischbar und wirkt viskositätsreduzierend und somit verlaufsfördernd. Des Weiteren katalysiert Benzylalkohol die Härtungsreaktion von aminischen Härtern und Epoxidharzen. Zudem unterdrückt Benzylalkohol die unerwünschte Carbamat-Bildung, welche als Nebenreaktion des Amins mit dem Kohlendioxid der Umgebungsluft einhergeht.

Für die Nutzung von Benzylalkohol in Epoxidharzbeschichtungen sind zukünftig deutliche Einschränkungen zu erwarten. Diese ergeben sich unter anderem aus verschiedenen nationalen und internationalen Richtlinien (EU-Decopaint-Richtlinie) zur Begrenzung von VOC-Emissionen (eng.: volatile organic compound = deutsch: flüchtige organische Verbindungen) aus Beschichtungsstoffen und zur Reduzierung des Gesundheitsrisikos des Verarbeiters und Nutzers durch flüchtige und schwerflüchtige Verbindungen (VOC und SVOC) (siehe Forderungen des Ausschusses für die gesundheitliche Bewertung von Bauprodukten = AgBB) oder die Zertifizierung von Gebäuden gemäß der Deutschen Gesellschaft für Nachhaltiges Bauen e.V. (DGNB) oder der Leadership in Energy & Environmental Design (LEED).

Hieraus ergibt sich ein hoher Bedarf an der Entwicklung von neuen Epoxysystemen, die frei von Benzylalkohol sind.

Aus dem Stand der Technik sind einige Lösungsansätze zu dieser Fragestellung bekannt.

In dem Übersichtsartikel "Ohne Benzylalkohol geht's auch" aus Farbe und Lacke, 2010, 3, werden einige Hilfsstoffe genannt, wie beispielsweise spezielle Polyamidoaminhärter, spezielle niedrigviskose, hochsiedende Harze oder auch hochsiedende Glykolether, um härtbare Zusammensetzung ohne Benzylalkohol zu formulieren. Prinzipiell ist es möglich, Hilfsstoffe einzusetzen, jedoch ist dies mit viel Aufwand und hohen Kosten verbunden.

EP 2706076 B1 offenbart Benzylalkohol-freie Epoxidharz-basierende Zusammensetzungen mit einem maßgeschneiderten Furfurylamin-basierten Härter. Durch seinen aufwendigen Herstellungsprozess ist er jedoch teurer als übliche aminische Härter. Neben dem ökonomischen Aspekt wird sicherlich auch diesem "speziellen" Aminhärter der kommerzielle Durchbruch erschwert, da in etablierten Systemen der Aminhärter nicht ohne weiteres ausgetauscht werden kann.

Benzylalkohol-basierte Alkoxylate sind dem Fachmann bekannt. So werden Benzylglykole in der WO 2005026275 und US 8129032 B2 als Additiv in wässrigen Dispersionen beschrieben. Auch die Verwendung als Dispergiermittel für Farbpigmente (JP4787416 B2, US 20060001011) oder als Emulgator in wässrigen Epoxy-basierten Dispersionen (JP 60019774 B, JP 60011728 B und JP 51033940 B) ist bekannt.

Benzylalkohol-basierte Propoxylate werden in der WO 9937714 A1 im Kontext von lösemittelhaltigen und lösemittelfreien Epoxyharz-Systemen beschrieben. Darin werden Phenol- und Benzylalkohol-basierte Propoxylate als Weichmacher für Epoxidharz und Aminhärter beschrieben. Werden derartige Alkoxylate als Ersatz für Benzylalkohol in härtbaren Zusammensetzungen eingesetzt, so verlängert sich die Gelzeit, wobei die Oberflächenhärte des ausgehärteten Materials nach 5 d nahezu unbeeinflusst bleibt.

Es ist daher wünschenswert, ein Modifizierungsmittel für härtbare Zusammensetzungen auf Epoxidharzbasis bereitzustellen, wobei diese härtbaren Zusammensetzungen eine Reduzierung des VOC (volatile organic content) und SVOC (semi volatile organic content)-Wertes gegenüber vergleichbaren benzylalkoholhaltigen härtbaren Zusammensetzungen auf Epoxidharzbasis aufweisen, gleichzeitig keine signifikante Verschlechterung der verarbeitungsrelevanten Eigenschaften der härtbaren Zusammensetzung aufzeigen.

Zur Lösung der Aufgabe wird ein Modifizierungsmittel der eingangs genannten Art vorgeschlagen, wobei das Benzylalkohol-basierte Alkoxylat durch Umsetzung von Benzylalkohol mit Alkylenoxiden erhalten wird und mindestens ein Ethoxy-Fragment aufweist.

Unter Modifizierungsmittel wird im Rahmen dieser Erfindung folgendes verstanden: Es ist ein wichtiger Bestandteil in einer härtbaren Zusammensetzung, der positiv die Reaktivität und den Verlauf der Zusammensetzung beeinflusst. Im weitesten Sinne soll das erfindungsgemäße Modifizierungsmittel die gleiche Funktion wie der aus dem Stand der Technik bekannte Benzylalkohol für diesen Zweck darstellen. Benzylalkohol ist sowohl mit dem Epoxidharz als auch mit dem Aminhärter mischbar und wirkt viskositätsreduzierend und somit verlaufsfördernd. Des Weiteren katalysiert Benzylalkohol die Härtungsreaktion und den Umsatz von aminischen Härtern und Epoxidharzen. Der unerwünschten Bildung von Carbamat, als Nebenreaktion des nicht umgesetzten Amins mit dem Kohlendioxid der Umgebungsluft, wird damit durch den Einsatz von Benzylalkohol entgegengewirkt.

Es wurde nun gefunden, dass die mit dem erfindungsgemäßen Modifizierungsmittel hergestellte härtbare Zusammensetzung ein geringeres Ausgasungsverhalten aufweist als härtbare Zusammensetzungen mit Benzylalkoholen.

Das Ausgasungsverhalten wird anhand des flüchtigen Anteils bis zu einem Siedepunkt von 365°C abgeleitet. Bis zu diesem Bereich werden die flüchtigen Bestandteile (VOC) und schwerflüchtigen Anteile (SVOC) als Summenparameter erfasst. Je niedriger der Prozentwert ist, desto weniger flüchtige Substanzen werden in die Umgebung abgegeben. Die flüchtigen Anteile wurden per Gaschromatographie angelehnt an DIN EN ISO 11890-2 bestimmt. 365 °C ist dabei die Grenze des SVOC (semi volatile oranic content, mittelflüchtiger organischer Anteil).

Ebenso konnte überraschend festgestellt werden, dass das erfindungsgemäße Modifizierungsmittel im Hinblick auf die verarbeitungsrelevanten Eigenschaften, wie z. B. Verdünnungswirkung, Viskositätsreduzierung und Härtungsreaktion, keine signifikante Verschlechterung gegenüber den üblichen Benzylalkoholen aufweist.

Gegenüber Benzylalkohol-basierten Propoxylaten zeigt das erfindungsgemäße Modifizierungsmittel zudem eine Verbesserung in mindestens einer verarbeitungsrelevanten Eigenschaft bei gleicher Alkoxyfragment-Kettenlänge.

Beim Einsatz der erfindungsgemäßen härtbaren Zusammensetzungen, härtbare Zusammensetzung mit dem erfindungsgemäßen Modifizierungsmittel, konnte insbesondere eine deutlich verbesserte Viskositätsreduktion im Vergleich zur Verwendung der Benzylalkohol-basierten Propoxylate festgestellt werden. Ebenso war eine schnellere Aushärtung, eine bessere Fließfähigkeit und eine geringere Neigung zur Carbamatbildung der härtbaren Zusammensetzungen zu verzeichnen.

Werden in der vorliegenden Erfindung chemische (Summen-)Formeln verwendet, so können die angegebenen Indizes sowohl absolute Zahlen als auch Mittelwerte darstellen.

Bei polymeren Verbindungen stellen die Indizes vorzugsweise Mittelwerte dar.

Bei Prozentangaben handelt es sich, wenn nicht anders angegeben, um Angaben in Gewichtsprozent.

Sind nachfolgend Messwerte angegeben, so sind diese Messungen, wenn nicht anders angegeben, bei Normalbedingungen (25 °C und 1013 mbar) durchgeführt worden.

Werden nachfolgend Mittelwerte angegeben, so handelt es sich, wenn nicht anderes angegeben, um Gewichtsmittel.

Vorzugsweise handelt es sich bei den erfindungsgemäßen Benzylalkohol-basierten Alkoxylaten um Benzylalkohol-basierte Ethoxylate.

Vorzugsweise handelt es sich bei den erfindungsgemäßen Benzylalkohol-basierten Alkoxylaten um Benzylalkohol-basierte Mischalkoxylate gemäß der Formel (I): 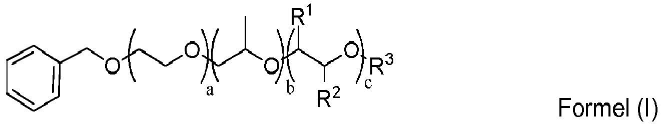 wobei
- a = Alkoxy-Fragment (a) =: 1 bis 10, bevorzugt 2 bis 7, insbesondere bevorzugt 3 bis 6,
- b = Alkoxy-Fragment (b) =: 0 bis 10, bevorzugt 1 bis 7, insbesondere bevorzugt 1 bis 5,
- c = Alkoxy-Fragment (c) =: 0 bis 10, bevorzugt 0 bis 6, insbesondere bevorzugt 0 bis 3,
- R¹, R² =: unabhängig voneinander Wasserstoff, eine Alkylgruppe mit 2 bis 20 Kohlenstoffatomen, eine Aryl- oder Alkarylgruppe, bevorzugt Ethyl-, Octyl-, Decyl-, Phenyl-, insbesondere bevorzugt Ethyl- oder Phenyl-, mit der Maßgabe, dass R¹ ungleich H, wenn R² gleich Methyl, und dass nicht beide Reste R¹ und R² gleichzeitig H sein dürfen,
- R³ =: unabhängig voneinander ein Wasserstoffradikal, eine Acetyl-, eine Phosphorester- oder eine Alkylgruppe mit 1 bis 20 Kohlenstoffatomen, die gegebenenfalls auch weiter substituiert sein kann, bevorzugt eine Acetyl-, Methyl-, Phosphorestergruppe oder ein Wasserstoffradikal, besonders bevorzugt ein Wasserstoffradikal.

Vorzugsweise wird das erfindungsgemäße Modifizierungsmittel durch Umsetzung von Benzylalkohol mit Alkylenoxiden hergestellt.

Bei Benzylalkohol (CAS: 100-51-6) handelt es sich um einen aromatischen Alkohol, der auch unter den chemischen Bezeichnungen Phenylmethanol und Phenylcarbinol bekannt ist. Es handelt sich um einen natürlichen Rohstoff, welcher zu ca. 6% in Jasminblütenöl, aber auch in Nelkenöl oder Goldlacköl enthalten ist.

Generell können alle dem Fachmann bekannten Alkylenoxide eingesetzt werden. Vorzugsweise werden beispielsweise Ethylenoxid (EO), Propylenoxid (PO), 1,2-Epoxy-2-methylpropan (Isobutylenoxid), Epichlorhydrin, 2,3-Epoxy-1-propanol, 1,2-Epoxybutan (Butylenoxid, im Folgenden auch als BO abgekürzt), 2,3-Epoxybutan, 2,3-Dimethyl-2,3-epoxybutan, 1,2-Epoxypentan, 1,2-Epoxy-3-methylpentan, 1,2-Epoxyhexan, 1,2-Epoxycyclohexan, 1,2-Epoxyheptan, 1,2-Epoxyoctan, 1,2-Epoxynonan, 1,2-Epoxydecan, 1,2-Epoxyundecan, 1,2-Epoxydodecan, Styroloxid (im Folgenden auch als SO abgekürzt), 1,2-Epoxycyclopentan, 1,2-Epoxycyclohexan, Vinylcyclohexenoxid, (2,3-Epoxypropyl)benzol, Vinyloxiran, 3-Phenoxy-1,2-epoxypropan, 2,3-Epoxymethylether, 2,3-Epoxyethylether, 2,3-Epoxylisopropylether, (3,4-Epoxybutyl)stearat, 4,5-Epoxypentylacetat, 2,3-Epoxylpropanmethacrylat, 2,3-Epoxylpropanacrylat, Gylcidylbutyrat, Methylglycidat, Ethyl-2,3-epoxybutanoat, 4-(Trimethylsilyl)butan-1,2-epoxid, 4-(Triethylsilyl)butan-1,2-epoxid, 3-(Perfluoromethyl)-1,2-epoxypropan, 3-(Perfluoroethyl)-1,2-epoxypropan, 3-(Perfluorobutyl)-1,2-epoxypropan, 3-(Perfluorohexyl)-1,2-epoxypropan, 4-(2,3-Epoxypropyl)morpholin, 1-(Oxiran-2-ylmethyl)pyrrolidin-2-on eingesetzt.

Alle genannten Alkylenoxide können einzeln oder in beliebigen Mischungen zur Alkoxylierung des Benzylalkohols eingesetzt werden.

Besonders bevorzugt werden Ethylenoxid, Propylenoxid, Butylenoxid und Styroloxid eingesetzt.

Um beispielsweise die in Formel (I) angegebenen Alkoxy-Fragmente (a) und (b) in das erfindungsgemäße Modifizierungsmittel einzubringen, können für das Alkoxy-Fragment (a) bevorzugt Ethylenoxid (EO) und für das Alkoxy-Fragment (b) bevorzugt Propylenoxid (PO), auch unter der Bezeichnung 1,2-Epoxypropan bekannt, eingesetzt werden.

Um beispielsweise die in Formel (I) angegebenen Alkoxy-Fragmente (c) in das erfindungsgemäße Modifizierungsmittel einzubringen, können für das Alkoxy-Fragment (c) bevorzugt Butylenoxid (BO) und/oder Styroloxid (SO) eingesetzt werden.

In einer ganz besonders bevorzugten Ausführungsform werden Ethylenoxid und Propylenoxid in einem Molaren-Verhältnis von 1 : 10 bis 10 : 0, bevorzugt von 3 : 1 bis 1 : 0, besonders bevorzugt 3:3, 3:1, 4:0 oder 1:1, eingesetzt.

Die Alkoxy-Fragmente (a), (b), (c) können vorzugsweise eine statistische Verteilung und/oder blockartige Verteilung aufweisen.

Die erforderliche Verteilung der Alkoxy-Fragmente (a) und/oder (b) und/oder (c) kann durch die jeweilige dem Fachmann bekannte Verfahrensführung, insbesondere durch die Zugabe-Reihenfolge bzw. die gleichzeitige Zugabe der jeweiligen Alkylenoxide, (einzeln oder als Gemisch), und durch deren molares Verhältnis zueinander erreicht werden.

Die Alkoxylierung des Benzylalkohols kann sowohl unter Basen-, Säuren- oder Übergangsmetall-Katalyse erfolgen. Bevorzugt wird die Alkoxylierung in Anwesenheit von Doppelmetallcyanid-(DMC)-Katalysatoren oder Basen durchgeführt. Besonders bevorzugt wird die Alkoxylierung in Anwesenheit von Basen durchgeführt.

Die Basen-katalysierte Alkoxylierung von Benzylalkohol wird vorzugsweise mit Alkalimetallhydroxid oder -alkoxid, vorzugsweise Natriummethylat, Kaliummethylat oder Kaliumhydroxid, besonders bevorzugt Kaliummethylat oder Kaliumhydroxid, zumindest teilweise durchgeführt. Die eingesetzte Menge an Alkalimetallhydroxiden oder - alkoxiden beträgt vorzugsweise von 1 bis 15 mol-%, bevorzugt von 1,5 bis 10 mol-%, besonders bevorzugt 2 bis 7 mol-%.

Die Alkoxylierung wird vorzugsweise bei einer Temperatur zwischen 80°C und 200°C, bevorzugt von 90°C bis 170°C und besonders bevorzugt von 100 bis 125°C durchgeführt. Die Umsetzung erfolgt vorzugsweise bei Drücken im Bereich von 0,001 bis 100 bar, bevorzugt im Bereich von 0,005 bis 10 bar und ganz besonders bevorzugt von 0,01 bis 5 bar (jeweils absolute Drücke). Gegebenenfalls kann die Alkoxylierung auch in Gegenwart eines Inertgases (z.B. Stickstoff) durchgeführt werden.

Die terminalen Hydroxygruppen der Benzylalkohol-Alkoxylate können bevorzugt frei bleiben oder können teilweise oder komplett modifiziert werden, um die optimale Kompatibilität in der späteren Anwendungsmatrix einstellen zu können.

Demnach weist das Benzylalkohol-basierte Alkoxylat bevorzugt eine terminale Hydroxygruppe auf.

Als Modifikation sind Umesterungen, Veresterungen oder Veretherungen ebenso denkbar wie weitere Kondensations- oder Additionsreaktionen mit z. B. Isocyanaten, die nach beliebigen Verfahren des Stands der Technik durchgeführt werden können.

Vorzugsweise werden die terminalen Hydroxygruppen acetyliert, methyliert oder phosphoryliert, besonders bevorzugt phosphoryliert.

Bevorzugt beträgt der Alkoxylierungsgrad 3 bis 10, besonders bevorzugt 4 bis 8.

Die Polydispersität (Mw/Mn) der Benzylalkohol-Alkoxylate der Formel (I), bestimmt mittels GPC, beträgt vorzugsweise < 2,5, bevorzugt < 2,0 und besonders bevorzugt von >1,05 bis <1,5.

Ein weiterer Gegenstand der Erfindung ist die Verwendung von Benzylalkohol-basierten Alkoxylaten, wobei das Benzylalkohol-basierte Alkoxylat mindestens ein Ethoxy-Fragment aufweist, als Modifizierungsmittel für härtbare Zusammensetzungen enthaltend mindestens ein Epoxidharz, mindestens einen Härter, vorzugsweise einen aminischen Härter oder einen sauren Härter, und optional weitere Hilfskomponenten.

Vorzugsweise werden die erfindungsgemäßen Benzylalkohol-basierten Alkoxylate gemäß Formel (I) als Modifizierungsmittel verwendet.

Die erfindungsgemäßen Benzylalkohol-Alkoxylate der Formel (I) können für vielfältige Anwendungen eingesetzt werden.

Ebenfalls Gegenstand der vorliegenden Erfindung sind härtbare Zusammensetzungen enthaltend
(1) mindestens ein Epoxidharz,
(2) mindestens einen Härter, (2a) aminischer Natur oder (2b) saurer Natur,
(3) mindestens ein Modifizierungsmittel gemäß Formel (I),
(4) gegebenenfalls weitere Hilfskomponenten.

Als Epoxidharze, Komponente (1) der härtbaren Zusammensetzungen, können prinzipiell jegliche Epoxidharze eingesetzt werden, die mit Aminen ausgehärtet werden können. Im Wesentlichen handelt es sich bei Epoxidharzen um Prepolymere, die pro Molekül zwei oder mehr Epoxidgruppen enthalten. Am weitesten verbreitet sind Bisphenol-basierte Glycidylether oder Novolake, welche meist Viskositäten von größer 10 Pa*s aufweisen.

Beispielsweise seien Bisphenol A-diglycidylether, Bisphenol F-diglycidylether oder cycloaliphatische Typen wie z. B. 3,4-Epoxycyclohexyl-epoxyethan oder 3,4-Epoxycyclohexylmethyl-3,4-epoxy-cyclohexancarboxylat genannt.

Vorzugsweise werden Bisphenol A-basierte Epoxidharze und Bisphenol F-basierte Epoxidharze in der erfindungsgemäßen Zusammensetzung eingesetzt.

Als aminische Härter, Komponente (2a) der härtbaren Zusammensetzungen, werden üblicherweise aliphatische, cycloaliphatische, araliphatische oder aromatische Amine oder Polyamine eingesetzt. Bevorzugt werden aminhaltige Härter mit mindestens zwei oder mehr primären und/oder sekundären Aminogruppen eingesetzt. Beispielsweise seien für aliphatische Amine Diaminoethan, Diaminopropan, Diaminobutan, Neopentandiamin, Diaminohexan, sowie Bis(aminocyclohexyl)methan, Diaminocyclohexan, 3,3'-Dimethyl-4,4'-diaminodicyclohexylmethan, Tricyclododecandiamin, Norbornandiamin, TCD-diamin, N-Aminoethylpiperazin, Isophorondiamin, 1,3- und/oder 1,4-Bis(aminomethyl)cyclohexan, Trimethyl-hexamethylendiamin, N-Aminoethylpiperazin, 1,4-Bis(aminopropyl)piperazin genannt.

Als aromatische Amine können vorzugsweise Methylendianilin, Xylylendiamin, m-Phenylenbis(methylamin) eingesetzt werden. Als Polyetheramine können vorteilhaft Polyoxyalkylenamine wie Diethylentriamin, Triethylentetramin, Tetraethylentetraamin, usw. sowie Dipropylentriamin, Tripropylentetraamin, Polyaminoamide, und Umsetzungsprodukte von Aminen mit Acrylnitril und Mannichbasen genannt werden.

Als saure Härter, Komponente (2b) der härtbaren Zusammensetzung, werden üblicherweise Säuren und Säureanhydride eingesetzt. Bevorzugt werden Carbonsäureanhydride oder polymere Carbonsäureanhydride oder Carbonsäuranhydride enthaltende Polymere oder Harze verwendet. Beispielsweise seien Phthalsäureanhydrid, Tetrahydrophthalsäureanhydrid, Hexahydrophthalsäureanhydrid, Methyltetrahydrophthalsäureanhydrid, 3,6-Endomethylen Tetrahydrophthalsäureanhydrid, Hexachloroendomethylentetrahydrophthalsäureanhydrid, Methyl-3,6-endomethylentetrahydrophthalsäureanhydrid, Trimellitsäureanhydrid, Maleinsäureanhydrid, Acrylsäureanhydrid, Methacrylsäureanhydrid, Hydroxymethylacrylsäureanhydrid, Hydroxyethylacrylsäureanhydrid, Hydroxypropylacrylsäureanhydrid genannt.

Als Modifizierungsmittel Komponente (3) der härtbaren Zusammensetzungen, kann vorzugsweise eine Verbindung der Formel (I) eingesetzt werden.

Als Hilfskomponenten, Komponente (4) der härtbaren Zusammensetzungen, kann jegliche Komponente zugesetzt werden, die die Eigenschaften der erfindungsgemäßen Zusammensetzung positiv beeinflusst. Es können unabhängig voneinander ein oder mehrere Hilfskomponenten zugesetzt werden.

Im Weiteren werden einige Hilfskomponente aufgeführt, die für die erfindungsgemäße härtbare Zusammensetzung verwendet werden können. Die Aufzählung ist nicht abschließend.

Als Hilfskomponente sind beispielsweise Reaktivverdünner vorteilhaft einsetzbar. Unter dem Begriff Reaktivverdünner wird im Rahmen dieser Erfindung ein relativ niedrigmolekularer, niedrigviskoser Glycidylether verstanden, welcher kompatibel mit den anderen Zusammensetzungsbestandteilen ist. Bevorzugt eingesetzt werden können beispielsweise Butylglycidylether, Ethylhexylglycidylether, Phenylglycidylether, Glycidylether der Versatic-Säure, C12/C14-Glycidylether, C13/C15-Glycidylether, p-tert-Butyl-Phenylglycidylether, 1,6-Hexandiglycidylether, 1,4-Butandiglycidylether, Cyclohexandimethyldiglycidylether, Polypropylendiglycidylether, Polyethylendiglycidylether, Neopentlyglykoldiglycidylether, Glycerintriglycidylether, Trimethylolpropantriglycidylether sowie Kresylglycidylether.

Als Hilfskomponente sind beispielsweise Lösungsmittel vorteilhaft einsetzbar. Beispielsweise sind Xylol, Hyblen (CAS-Nr. 67774-74-7) oder iso-Propanol zu nennen. Der Einsatz von Lösungsmitteln in der erfindungsgemäßen Zusammensetzung ist jedoch weniger bevorzugt.

Als Hilfskomponente sind beispielsweise Katalysatoren wie organische Säuren oder tertiäre Amine vorteilhaft einsetzbar. Beispielsweise sind Salicylsäure, Tris-(N,N-dimethylaminomethyl)-phenol, Aminoethylpiperazin, zu nennen.

Als Hilfskomponente sind beispielsweise Füllstoffe vorteilhaft einsetzbar. Beispielsweise sind Sand, Silikate, Graphit, Talk, Siliciumdioxid zu nennen.

Als weitere Hilfskomponenten sind beispielsweise Weichmacher, Farbstoffe, Pigmente, Stabilisatoren, Verschnittharze, Entlüfter, Netz- und Dispergiermittel, Oberflächenadditive, Substratnetzmittel, ESD-Additive (ESD = electrostatic discharge) usw. vorteilhaft einsetzbar.

In den erfindungsgemäßen härtbaren Zusammensetzungen können auch beliebige Mischungen von unterschiedlichen Epoxidharzen (1) und/oder Aminhärtern (2a) vorteilhaft eingesetzt werden.

In den erfindungsgemäßen härtbaren Zusammensetzungen können auch beliebige Mischungen von unterschiedlichen Epoxidharzen (1) und/oder sauren Härtern (2b) vorteilhaft eingesetzt werden.

In einer besonderen Ausführungsform der vorliegenden Erfindung kann es auch vorteilhaft sein, Prepolymere von Epoxidharzen und Aminhärtern allein, oder in Abmischung mit weiterem Epoxidharz und/oder Aminhärter in den erfindungsgemäßen Zusammensetzungen einzusetzen.

Beim Einsatz des erfindungsgemäßen Modifizierungsmittels (3) ist dem Anwender jegliche Freiheit gegeben. Das erfindungsgemäße Modifizierungsmittel (3) kann bei der Herstellung der härtbaren Zusammensetzung zugegeben werden. Es ist auch möglich vorab gefertigte Mischungen aus dem erfindungsgemäßen Modifizierungsmittel (3) mit dem Epoxidharz (1) zu machen. Zudem ist es auch möglich das erfindungsgemäße Modifizierungsmittel (3) mit dem Aminhärter (2a) oder dem sauren Härter (2b) vorzumischen. Alternativ können statt monomeren Harzen oder Härtern auch beliebige daraus hergestellte Prepolymere mit dem erfindungsgemäßen Modifizierungsmittel (3) gemischt werden. Der Zusatz der Hilfskomponente (4) ist sowohl zum Epoxidharz (1) als auch dem Härter (2a) oder (2b) sowie dem erfindungsgemäßen Modifizierungsmittel (3) als auch zu beliebigen Mischungen der zuvor genannten Komponenten möglich. In einer bevorzugten Ausführungsform wird das erfindungsgemäße Modifizierungsmittel (3) dem Epoxidharz (1) oder den jeweiligen Härtern (2a) oder (2b) oder bei der Herstellung der härtbaren Zusammensetzung zugesetzt, besonders bevorzugt den Härtern (2a) oder (2b) oder bei der Herstellung der härtbaren Zusammensetzung.

Für eine anwendungstechnisch vorteilhafte Zusammensetzung kann ein besonderes Molverhältnis der Epoxidgruppen des Epoxidharzes (1) zu den Aminogruppen des Härters (2a) erforderlich sein. In einer bevorzugten Ausführungsform der erfindungsgemäßen Zusammensetzung beträgt das Molverhältnis der Epoxidgruppen aller Epoxidharze (1) in der Zusammensetzung zu den Aminogruppen aller aminischen Härtern (2a) bevorzugt zwischen 1 : 0,5 und 1 : 1,5, besonders bevorzugt 1 : 0,7 und 1 : 1,3, insbesondere bevorzugt 1 : 0,9 und 1 : 1,1.
Als Mischungsverhältnisse der Zusammensetzung aus Epoxidharz (1), aminischem Härter (2a) oder saurem Härter (2b), dem erfindungsgemäßen Modifizierungsmittel (3) und weiteren Hilfskomponenten (4) werden bezogen auf das Gesamtgewicht des Epoxidharzes (1) und des aminischen Härters (2a) in der bevorzugten Ausführungsform ein Massenverhältnis zum erfindungsgemäßen Modifizierungsmittel (3) bevorzugt zwischen 1 : 0 und 1 : 1, besonders bevorzugt zwischen 1 : 0 und 1 : 0,5, insbesondere bevorzugt zwischen 1 : 0 und 1 : 0,2 eingesetzt. Das Mischungsverhältnis bezogen auf das Gesamtgewicht des Epoxidharzes (1) und des aminischen Härters (2a) zu weiteren Hilfskomponenten (4) beträgt bevorzugt zwischen 1 : 0 und 1 : 15, besonders bevorzugt 1 : 0 und 1 : 8, insbesondere bevorzugt 1 : 0 und 1 : 1.

Die erfindungsgemäßen Zusammensetzungen werden insbesondere vorteilhaft in Fußbodenbeschichtungen, Schutzbeschichtungen, Composites, Klebern und Dichtmassen im Industriebau, Lager und Logistik, Hoch- und Verwaltungsbau, Gewässerschutz, Nahrungsmittelindustrie, Reinräumen, Parkbauten, Korrosionsschutz, Betonschutz, Bauwerksabdichtung, Hygienebeschichtung, anderen industriellen Beschichtungen und Wandanstrichen eingesetzt. Eingeschlossen sind auch Anwendungen in denen die Beschichtungen zusätzliche Funktionalitäten und Eigenschaften haben, wie z. B. Schutz vor Korrosion, Ableitfähigkeit, Brandschutz.

Die erfindungsgemäßen Gegenstände werden nachfolgend beispielhaft beschrieben, ohne dass die Erfindung auf diese beispielhaften Ausführungsformen beschränkt sein soll.

### Messmethoden:

Zur Bestimmung von Parametern oder Messwerten werden vorzugsweise die nachfolgend beschriebenen Methoden verwendet. Insbesondere wurden diese Methoden in den Beispielen des vorliegenden Schutzrechts verwendet.

Gewichtsmittlere und zahlenmittlere Molekulargewichte werden im Rahmen dieser Erfindung für die hergestellten Benzylalkohol-Alkoxylate der Formel (I) kalibriert gegen einen Polypropylenglykolstandard durch Gelpermeationschromatographie (GPC) bestimmt. Die GPC wurde durchgeführt auf einem Agilent 1100 ausgestattet mit einem RI-Detektor und einer SDV 1000/10000 Å Säulenkombination bestehend aus einer 0,8 cm x 5 cm Vorsäule und zwei 0,8 cm x 30 cm Hauptsäulen bei einer Temperatur von 30°C und einer Fließrate von 1 mL/min (mobile Phase: THF). Die Probenkonzentration betrug 10 g/L und das Injektionsvolumen 20 µL.
Der Polydispersitätsindex (PDI) ist der Quotient aus M_{w} durch Mₙ (PDI = M_{w}/Mₙ).

Die flüchtigen Anteile wurden per Gaschromatographie angelehnt an DIN EN ISO 11890-2 bestimmt. Vor der Messung wurden die alkoholischen End-Gruppen durch Derivatisierung mit *N*-Methyl-*N*-trifluoroacetamide (MSTFA) in die korrespondierenden Trimethylsilylether überführt.
Die Analyse erfolgte mittels Gaschromatographie ausgestattet mit on-column Injektion und FID Detektion. Die Bestandteile wurden an einer apolaren Trennsäule (DB-5 HT; Länge 30m; Durchmesser 0,25 mm; Filmdicke 0,1 µm, Temperaturprogramm 65 °C bis 365 °C mit 10 °C pro Minute; anschließend 15 Minuten bei 365 °C Haltezeit) aufgetrennt.
Zur Quantifizierung wurde die Summe der Peakflächen der als VOC/SVOC eingestuften Bestandteile im Vergleich zu der Gesamt-Peakfläche aller in der Probe detektierten Substanzen bestimmt (Flächen-% Auswertung).

Die nasschemische Analytik wurde in Anlehnung an internationale Standardmethoden durchgeführt: Iodzahl (IZ; DGF C-V 11 a (53); Säurezahl (SZ; DGF C-V 2); OH-Zahl (ASTM D 4274 C).

Der Gehalt der primären OH-Kettentermini der Polyether wurde durch die Auswertung quantitativer ¹³C-NMR-Spektren bestimmt. Hierzu wurde die Intensität der Signale bei einer Verschiebung von ∼62 ppm (primäre OH-Gruppen) mit den Signalen bei ∼67 ppm (sekundäre OH-Gruppen) ins Verhältnis gesetzt.
Die NMR-Spektren wurden mit einem 400 MHz Spektrometer der Firma Bruker unter Einsatz eines 5 mm QMP-Kopfes gemessen. Es wurden quantitative NMR-Spektren in Gegenwart eines geeigneten Beschleunigungsagenzes gemessen. Die zu untersuchende Probe wurde in einem geeigneten deuterierten Lösungsmittel (Methanol) gelöst und in 5 mm oder ggf. 10 mm NMR-Röhrchen überführt.

### Beispiel 1:

### Synthese von erfindungsgemäßen Modifizierungsmitteln und Benzylalkohol-basierten Propoxylaten (Beispiele M1 - M14, VG1 und VG 2)

In einem 5 Liter Autoklaven wird die entsprechende Menge Benzylalkohol gemeinsam mit 5 mol-% Kaliummethylat unter Stickstoff vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 100 °C erhitzt und auf ca. 100 mbar evakuiert, um das Methanol aus dem Katalyseschritt zu entfernen. Dann wurde die Temperatur auf 120°C erhöht und das/die Alkylenoxid(e) dergestalt zudosiert, dass sich die in Tabelle 1 angegebene Verteilung der Alkoxy-Fragmente ergab.

Die Dosagegeschwindigkeit des bzw. der Alkylenoxid(e) wurde dabei so gewählt, dass der Druck im Reaktor nicht über 2 bar anstieg. Nach beendeter Dosage wurde zunächst gewartet bis der Druck nicht weiter sank, was als Zeichen eines nahezu quantitativen Umsatzes des bzw. der Alkylenoxid(e) gewertet wurde. Zur Vervollständigung des Alkylenoxidumsatzes wurde noch eine einstündige Nachreaktion durchgeführt. Wenn ein blockartiger Alkoxyaufbau angestrebt wird, so wird das zuvor beschriebene Vorgehen für jedes anzulagernde reine Alkylenoxid wiederholt. Wenn eine statistische Anlagerung der Alkylenoxide angestrebt wird, so wird eine homogene Mischung der jeweiligen Alkylenoxide zudosiert. Abschließend (nach Anlagerung des letzten Alkylenoxids bzw. Alkylenoxidgemisches mit entsprechender Nachreaktion) wurde das Reaktionsgemisch durch Anlegen eines Drucks (p < 20 mbar) desodoriert, um Spuren an nicht umgesetztem Alkylenoxid zu entfernen. Anschließend wurde das Benzylalkohol-basierte Alkoxylat mit verdünnter Phosphorsäure neutralisiert und mit 500 ppm ANOX 20 AM stabilisiert. Anschließend wurde das Wasser destillativ im Vakuum entfernt und die ausgefallenen Salze abfiltriert.

Es wurden in allen Fällen farblose bis gelbliche Benzylalkohol-basierte Alkoxylate erhalten, deren wesentlichen Kennzahlen in Tabelle 1 zusammengefasst sind. Der in den folgenden Tabellen beschriebene Aufbau erklärt sich folgendermaßen: Sind die Alkoxy-Fragmente (beispielsweise EO oder PO) durch ein "+" getrennt, handelt es sich um einen blockartigen Aufbau, sind die Alkoxy-Fragmente durch einen "/" getrennt, handelt es sich um einen statistischen Aufbau. Zur Vereinfachung der Darstellung in den Tabellen wurden die Alkoxy-Fragmente gemäß den eingesetzten Alkylenoxiden EO, PO, BO und SO bezeichnet.

### Beispiel 1a: Acetylierung des erfindungsgemäßen Modifizierungsmittels M1

Unter Schutzgas wurden in einem 4 Liter Dreihals-Kolben ausgestattet mit Tropftrichter und Rückflußkühler 1979 g des Modifizierungsmittels 1 zusammen mit katalytischen Mengen an konzentrierter Salzsäure vorgelegt und erwärmt. Dann wurde langsam Essigsäureanhydrid zugegeben. Nach kompletter Addition wurde das Gemisch noch 4 h bei 100°C gerührt. Dann wurden vorhandene Säurereste abdestilliert und man erhielt ein terminal acetyliertes Modifizierungsmittel M1Ac mit einer Säurezahl von 0,1 und einer Hydroxylzahl von 0 mg KOH/g.

In analoger Weise wurde auch das Modifizierungsmittel M5 acetyliert. Man erhielt ein terminal acetyliertes Modifizierungsmittel M5Ac mit einer Säurezahl von 0,1 und einer Hydroxylzahl von 0 mg KOH/g.

### Beispiel 1b: Methylierung des erfindungsgemäßen Modifizierungsmittels M1

Unter Schutzgas werden in einem 4 Liter Dreihals-Kolben ausgestattet mit Destillationsbrücke 1620 g des erfindungsgemäßen Modifizierungsmittels M1 vorgelegt und auf 50°C erwärmt. Bei dieser Temperatur werden langsam 130 mol-% Natriummethylat zugegeben. Das entstehende Methanol wird abdestilliert. Anschließend wird Wasserstrahlvakuum angelegt, die Temperatur auf 120°C erhöht und 1,5 h Methylchlorid mit Hilfe eines Gaseinleitungsrohres in die Lösung eingeleitet. Nach einem erneuten Vakuumdestillationsschritt wird erneut Methylchlorid über einen Zeitraum von 1 h eingeleitet. Dann wird destilliert, neutralisiert sowie filtriert und man erhält ein terminal methyliertes Modifizierungsmittel M1 Me mit einer Säurezahl von 0,1 und einer Hydroxylzahl von 1,0 mg KOH/g.

In analoger Weise wurde auch das Modifizierungsmittel M5 methyliert. Man erhielt ein terminal methyliertes Modifizierungsmittel M5Me mit einer Säurezahl von 0,1 und einer Hydroxylzahl von 2,4 mg KOH/g.

**Tabelle 1:**

| **Aufbau und physikalische Daten der Benzylalkohol-basierten Alkoxylate** | | | | |
|---|---|---|---|---|
| **Benzylalkoholbasierte Alkoxylate** | **Aufbau** | **SZ** | **OHZ** | **prim. OH-Termini** |
| | | [mg KOH/g] | | [%] |
| M1 | Benzylalkohol + 3 EO | 0,1 | 220 | 100 |
| M2 | Benzylalkohol + 6 EO | 0,1 | 147 | 100 |
| M3 | Benzylalkohol + 3 EO/3 PO | 0,2 | 128 | 14 |
| M4 | Benzylalkohol + 3 PO + 3 EO | 0,3 | 136 | 74 |
| M5 | Benzylalkohol + 2 EO/2 PO | 0,3 | 180 | 19 |
| M6 | Benzylalkohol + 1 EO/5 PO | 0,1 | 132 | 0 |
| M7 | Benzylalkohol + 2 EO/4 PO | 0,1 | 133 | 0 |
| M8 | Benzylalkohol + 1 EO/3 PO | 0,1 | 173 | 2 |
| M9 | Benzylalkohol + 1 PO + 3 EO | 0,1 | 186 | 71 |
| M10 | Benzylalkohol + 3 EO + 1 PO | 0,1 | 183 | 32 |
| M11 | Benzylalkohol + 1 EO/3 PO/2 BO | 0,2 | 118 | 0 |
| M12 | Benzylalkohol + 1 EO/3 PO/2 SO | 0,3 | 105 | 0 |
| M13 | Benzylalkohol + 2 EO/1 PO | 0,2 | 267 | 31 |
| M14 | Benzylalkohol + 1 EO/2 PO | 0,3 | 270 | 5 |
| M1Ac | Benzylalkohol + 3 EO, acetyliert | 0,1 | 0 | 0 |
| M5Ac | Benzylalkohol + 2 EO/2 PO, acetyliert | 0,1 | 0 | 0 |
| M1Me | Benzylalkohol + 3 EO, methyliert | 0,1 | 1 | 100 |
| M5Me | Benzylalkohol + 2 EO/2 PO, methyliert | 0,1 | 2,4 | 0 |
| VG1 (Vergleichsbeispiel) | Benzylalkohol + 3 PO | 0,2 | 194 | 0 |
| VG2 (Vergleichsbeispiel) | Benzylalkohol + 6 PO | 0,1 | 117 | 0 |

M1 bis M14 sind erfindungsgemäße Modifizierungsmitteln. VG1 und VG2 sind Vergleichsbeispiele.

### Beispiel 2:

### Ausgasungsverhalten

Das Ausgasungsverhalten des erfindungsgemäßen Modifizierungsmittels M1-14 bzw. des Benzylalkohols bzw. der Vergleichsbeispiele wird anhand des flüchtigen Anteils (VOC und SVOC = Flüchtige und Mittelflüchtige organische Verbindungen) in Anlehnung an die Definition der DIN EN 11890-2 abgeleitet. Die flüchtigen Anteile wurden per Gaschromatographie angelehnt an DIN EN ISO 11890-2 bestimmt. Vor der Messung wurden die alkoholischen End-Gruppen durch Derivatisierung mit *N-*Methyl-*N*-trifluoroacetamide (MSTFA) in die korrespondierenden Trimethylsilylether überführt. Die Analyse erfolgte mittels Gaschromatographie ausgestattet mit on-column Injektion und FID Detektion. Die Bestandteile wurden an einer apolaren Trennsäule (DB-5 HT; Länge 30m; Durchmesser 0,25 mm; Filmdicke 0,1 µm, Temperaturprogramm 65 °C bis 365 °C mit 10 °C pro Minute; anschließend 15 Minuten bei 365 °C Haltezeit) aufgetrennt. Zur Quantifizierung wurde die Summe der Peakflächen der als VOC/SVOC eingestuften Bestandteile im Vergleich zu der Gesamt-Peakfläche aller in der Probe detektierten Substanzen bestimmt (Flächen-% Auswertung). Je niedriger der Prozentwert ist, desto weniger flüchtige Substanzen werden in das Raumklima abgegeben.

**Tabelle 2:**

| **Flüchtiger organischer Anteil von erfindungsgemäßen Modifizierungsmitteln im Vergleich zu Benzylalkohol** | | |
|---|---|---|
| | **Aufbau** | **flüchtiger Anteil bis zu einem Siedepunkt von 365 °C** |
| | | % |
| Benzylalkohol | Benzylalkohol | 100 |
| M1 | Benzylalkohol + 3 EO | 58,6 |
| M2 | Benzylalkohol + 6 EO | 11,7 |
| M3 | Benzylalkohol + 3 EO/3 PO | 18,8 |
| M5 | Benzylalkohol + 2 EO/2 PO | 67,2 |
| M6 | Benzylalkohol + 1 EO/5 PO | 17,2 |
| M7 | Benzylalkohol + 2 EO/4 PO | 15,4 |
| M8 | Benzylalkohol + 1 EO/3 PO | 65,9 |
| M9 | Benzylalkohol + 1 PO + 3 EO | 60,0 |
| M10 | Benzylalkohol + 3 EO + 1 PO | 63,7 |
| M11 | Benzylalkohol + 1 EO/3 PO/2 BO | 18,4 |
| M12 | Benzylalkohol + 1 EO/3 PO/2 SO | 13,1 |
| M13 | Benzylalkohol + 2 EO/1 PO | 61,2 |
| M14 | Benzylalkohol + 1 EO/2 PO | 61,0 |
| M1Ac | Benzylalkohol + 3 EO, acetyliert | 52,3 |
| M5Ac | Benzylalkohol + 2 EO/2 PO, acetyliert | 61,6 |
| M1Me | Benzylalkohol + 3 EO, methyliert | 54,7 |
| M5Me | Benzylalkohol + 2 EO/2 PO, methyliert | 63,5 |
| VG1 | Benzylalkohol + 3 PO | 65,4 |
| VG2 | Benzylalkohol + 6 PO | 12,2 |

Alle erfindungsgemäßen Modifizierungsmittel sind Benzylalkohol im Ausgasungsverhalten überlegen. Es zeigt sich zudem, dass das erfindungsgemäße M1 gegenüber VG1 (gleiche Kettenlänge) und das erfindungsgemäße M2 gegenüber VG2 (gleiche Kettenlänge) weniger flüchtige organische Verbindungen abgeben.

### Beispiel 3:

### Viskositätsmessung des erfindungsgemäßen Modifizierungsmittels in Epoxidharz-Bindemitteln

Das erfindungsgemäße Modifizierungsmittel wird in Epoxidharz-Bindemitteln ohne Katalysator und in Epoxidharz-Bindemitteln mit Katalysator eingesetzt, um dessen Einfluss auf die Viskosität zu überprüfen.

**Formulierung I**

| | Anteil |
|---|---|
| Epikote 828, Bindemittel der Fa. Hexion | 20 g |
| M1, M1Ac, M1 Me, M2 - M5, M5Ac, M5Me, M6 - M10, M13, M14, VG1 - 2, Benzylalkohol | 2 g |

**Formulierung II**

| | Anteil |
|---|---|
| Epikote 828, Bindemittel der Fa. Hexion | 20 g |
| M1 - M3, M5 - M8, M13, M14, VG1 - 2, Benzylalkohol | 2 g |
| Salicylsäure (Katalysator) | 0,55 g |

Das Bindemittel wurde in PE-Bechern vorgelegt, das erfindungsgemäße Modifizierungsmittel bzw. Benzylalkohol bzw. Vergleichsbeispiele (VG) ggf. Katalysator zudosiert und die Formulierungen jeweils 1 min bei 1000 U/min in einem Speedmixer der Firma Hauschild eingearbeitet. Die Viskositäten der Formulierungen wurden mit einem Rheometer der Firma Anton Paar, Typ MCR 102 gemessen. Messparameter: Kegel/Platte CP 25/2, 23 °C, mehrere Messpunkte im Bereich von 1 -1000 1/s.

**Tabelle 3.1:**

| **Einfluss des erfindungsgemäßen Modifizierungsmittels auf die Viskosität der Formulierung I** | | | | |
|---|---|---|---|---|
| **Formulierung I mit** | **Aufbau** | **Viskosität bei 10 1/s** | **Viskosität bei 100 1/s** | **Viskosität bei 1000 1/s** |
| | | mPas | mPas | mPas |
| Benzylalkohol | Benzylalkohol | 1710 | 1720 | 1670 |
| M1 | Benzylalkohol + 3 EO | 3970 | 3990 | 3690 |
| M1Ac | Benzylalkohol + 3 EO, acetyliert | 3940 | 3980 | 3690 |
| M1Me | Benzylalkohol + 3 EO, methyliert | 3210 | 3000 | 2890 |
| M2 | Benzylalkohol + 6 EO | 4970 | 5040 | 4570 |
| M3 | Benzylalkohol + 3 EO/3 PO | 5650 | 5710 | 5100 |
| M4 | Benzylalkohol + 3 PO + 3 EO | 5877 | 5821 | 5067 |
| M5 | Benzylalkohol + 2 EO/2 PO | 4470 | 4510 | 4130 |
| M5Ac | Benzylalkohol + 2 EO/2 PO, acetyliert | 4330 | 4270 | 3950 |
| M5Me | Benzylalkohol + 2 EO/2 PO, methyliert | 3910 | 3580 | 3240 |
| M6 | Benzylalkohol + 1 EO/5 PO | 5620 | 5630 | 5010 |
| M7 | Benzylalkohol + 2 EO/4 PO | 4870 | 4890 | 4440 |
| M8 | Benzylalkohol + 1 EO/3 PO | 3610 | 3660 | 3430 |
| M9 | Benzylalkohol + 1 PO + 3 EO | 6104 | 5932 | 5186 |
| M10 | Benzylalkohol + 3 EO + 1 PO | 5726 | 5662 | 5034 |
| M13 | Benzylalkohol + 2 EO/1 PO | 3226 | 3270 | 3142 |
| M14 | Benzylalkohol + 1 EO/2 PO | 3163 | 3191 | 3073 |
| VG1 | Benzylalkohol + 3 PO | 4370 | 4410 | 4040 |
| VG2 | Benzylalkohol + 6 PO | 6280 | 6300 | 5520 |

**Tabelle 3.2:**

| **Einfluss des erfindungsgemäßen Modifizierungsmittels auf die Viskosität der Formulierung II** | | | | |
|---|---|---|---|---|
| **Formulierung II mit** | **Aufbau** | **Viskosität bei 10 1/s** | **Viskosität bei 100 1/s** | **Viskosität bei 1000 1/s** |
| | | mPas | mPas | mPas |
| Benzylalkohol | Benzylalkohol | 2650 | 2640 | 2510 |
| M1 | Benzylalkohol + 3 EO | 5310 | 5310 | 4750 |
| M2 | Benzylalkohol + 6 EO | 6670 | 6710 | 5820 |
| M3 | Benzylalkohol + 3 EO/3 PO | 7080 | 7110 | 6100 |
| M5 | Benzylalkohol + 2 EO/2 PO | 6140 | 6180 | 5440 |
| M6 | Benzylalkohol + 1 EO/5 PO | 7680 | 7670 | 6480 |
| M7 | Benzylalkohol + 2 EO/4 PO | 6850 | 6880 | 5950 |
| M8 | Benzylalkohol + 1 EO/3 PO | 6870 | 6900 | 6000 |
| M13 | Benzylalkohol + 2 EO/1 PO | 5180 | 5150 | 4520 |
| M14 | Benzylalkohol + 1 EO/2 PO | 5320 | 5360 | 4680 |
| VG1 | Benzylalkohol + 3 PO | 6260 | 6320 | 5530 |
| VG2 | Benzylalkohol + 6 PO | 7960 | 7950 | 6650 |

Zusammenfassung von Tabelle 3.1 und 3.2:
Vergleicht man das erfindungsgemäße Modifizierungsmittel M1 und VG1, welche die gleiche Kettenlänge haben, miteinander und analog die erfindungsgemäßen M2, M3, M6 und M7 mit dem VG2 (gleiche Kettenlänge), so sind die Modifizierungsmittel mit Ethoxy-Fragmenten den Vergleichsbeispielen mit reinem PO in der viskositätssenkenden Wirkung immer überlegen.

Zudem kann die viskositätssenkende Wirkung durch eine Endmodifikation verstärkt werden. Aus Tabelle 3.1 ist ersichtlich, dass die viskositätssenkende Wirkung der Formulierung I mit den erfindungsgemäßen M1Me bzw. M5Me etwas besser ist als die der Formulierung I mit den erfindungsgemäßen unmodifizierten M1 bzw. M5.

### Beispiel 4:

### Reaktionsgeschwindigkeit der Epoxidhärtung

### Beurteilung der Reaktivität von erfindungsgemäßen härtbaren Zusammensetzungen anhand der Viskositätsverdopplung

Die Verdopplung der Anfangsviskosität ist ein Maß für die Reaktionsgeschwindigkeit der Epoxidhärtungsreaktion.

**Zusammensetzung I**

| | Anteil |
|---|---|
| Epikote 828, Bindemittel der Firma Hexion | 20 g |
| M1, M2, M3, VG1-2, Benzylalkohol | 2 g |
| Vestamin IPD, Härter der Evonik Resource Efficiency GmbH | 4,54 g |

Das Bindemittel wurde in PE-Bechern vorgelegt, das erfindungsgemäße Modifizierungsmittel bzw. Benzylalkohol bzw. Vergleichsbeispiele zudosiert und die Mischungen jeweils 1 min bei 1000 U/min in einem Speedmixer der Firma Hauschild eingearbeitet. Der Härter wurde zugewogen und die härtbaren Zusammensetzungen 1 min bei 2000 U/min im Speedmixer gerührt. Die Viskositäten der härtbaren Zusammensetzungen wurden mit einem Rheometer der Firma Anton Paar, Typ MCR 102 gemessen. Messparameter: Kegel/Platte CP 25/2, 23 °C, konstante Scherrate von 100 1/s, Messdauer bis zur Verdopplung des Startwertes.

**Tabelle 4:**

| **Einfluss des erfindungsgemäßen Modifizierungsmittels in einer härtbaren Zusammensetzung I auf die Reaktivität anhand der Viskositätsverdopplung** | | | | | |
|---|---|---|---|---|---|
| **Zusammensetzung I mit** | **Aufbau** | **Viskosität Start** | **Viskosität Ende** | **Zeit** | **prim. OH-Termini** |
| | | mPas | mPas | s | % |
| Benzylalkohol | Benzylalkohol | 1490 | 2980 | 1790 | 100 |
| M1 | Benzylalkohol + 3 EO | 1720 | 3440 | 3100 | 100 |
| M2 | Benzylalkohol + 6 EO | 1710 | 3420 | 3350 | 100 |
| M3 | Benzylalkohol + 3 EO/3 PO | 1760 | 3520 | 3580 | 14 |
| VG1 | Benzylalkohol + 3 PO | 1720 | 3440 | 3900 | 0 |
| VG2 | Benzylalkohol + 6 PO | 1870 | 3520 | 3680 | 0 |

Vergleicht man das erfindungsgemäße Modifizierungsmittel M1 und VG1, beide haben die gleiche Kettenlänge, miteinander und die erfindungsgemäßen M2 und M3 mit dem VG2, so reagieren härtbare Zusammensetzungen mit den erfindungsgemäßen Modifizierungsmitteln mit Ethoxy-Fragmenten schneller als die mit reinen Propoxy-Fragmenten.

### Beispiel 5:

### Aushärtung

### Einfluss des erfindungsgemäßen Modifizierungsmittels in einer härtbaren Zusammensetzung auf die Aushärtung

Für die Prüfung der Shore D-Härte wurde die Rezeptur der härtbaren Zusammensetzung I eingesetzt.

Das Bindemittel wurde in PE-Bechern vorgelegt, das erfindungsgemäße Modifizierungsmittel zudosiert und die Mischungen jeweils 1 min bei 1000 U/min in einem Speedmixer der Firma Hauschild eingearbeitet. Der Härter wurde zugewogen und die härtbaren Zusammensetzungen 1 min bei 2000 U/min im Speedmixer gerührt. Daraus wurde eine Platte mit ca. 5 mm Schichtdicke gegossen. Es wurden die Shore D-Härten nach verschiedenen Intervallen gemessen.

**Tabelle 5:**

| **Entwicklung der Shore D Härte von erfindungsgemäßen härtbaren Zusammensetzungen** | | | |
|---|---|---|---|
| **Zusammensetzung I mit** | **Aufbau** | **Shore D Härte nach 2 Tagen** | **Shore D Härte nach 7 Tagen** |
| | | Shore | Shore |
| Benzylalkohol | Benzylalkohol | 86,3 | 88,6 |
| M1 | Benzylalkohol + 3 EO | 86,2 | 88,7 |
| M1Ac | Benzylalkohol + 3 EO, acetyliert | 83,9 | 88,9 |
| M1Me | Benzylalkohol + 3 EO, methyliert | 83,8 | 87,5 |
| M2 | Benzylalkohol + 6 EO | 84,4 | 88,4 |
| M3 | Benzylalkohol + 3 EO/3 PO | 84,5 | 85,9 |
| M5 | Benzylalkohol + 2 EO/2 PO | 85,1 | 88,1 |
| M5Ac | Benzylalkohol + 2 EO/2 PO, acetyliert | 83,2 | 88,0 |
| M5Me | Benzylalkohol + 2 EO/2 PO, methyliert | 83,6 | 87,9 |
| M6 | Benzylalkohol + 1 EO/5 PO | 85,2 | 87,6 |
| M7 | Benzylalkohol + 2 EO/4 PO | 85,0 | 87,5 |
| M8 | Benzylalkohol + 1 EO/3 PO | 82,3 | 87,1 |
| M9 | Benzylalkohol + 1 PO + 3 EO | 83,7 | 87,0 |
| M10 | Benzylalkohol + 3 EO + 1 PO | 85,9 | 87,0 |
| M11 | Benzylalkohol + 1 EO/3 PO/2 BO | 83,3 | 86,8 |
| M12 | Benzylalkohol + 1 EO/3 PO/2 SO | 82,6 | 85,9 |
| M13 | Benzylalkohol + 2 EO/1 PO | 86,5 | 89,6 |
| M14 | Benzylalkohol + 1 EO/2 PO | 87,2 | 89,6 |
| VG1 | Benzylalkohol + 3 PO | 78,6 | 88,7 |
| VG2 | Benzylalkohol + 6 PO | 81,6 | 87,4 |

Härtbare Zusammensetzungen mit den erfindungsgemäßen Modifizierungsmitteln zeigen vergleichbare Härteentwicklung nach 2 Tagen wie die Zusammensetzung mit Benzylalkohol. VG 1 und VG2 mit Propoxy-Fragmenten sind in der Anfangshärte deutlich unterlegen.

### Beispiel 6:

### Feuchtigkeitsempfindlichkeit

Als Maß für die Feuchtigkeitsempfindlichkeit einer Epoxy-Beschichtung wird die Carbamatbildungsneigung herangezogen. Dazu wird ein kleines Stück Schwamm mit Wasser getränkt, dieses auf die Beschichtung gelegt und ein Ochsenauge (Halbkugel aus Glas) darüber gestülpt. Die Oberfläche wird in Intervallen begutachtet. Das auf der Oberfläche gebildete Carbamat erscheint weißlich. Es wird auf einer Skala von 1 (sehr starke Carbamatbildung) bis 5 (keine Carbamatbildung) bewertet.

**Härtbare Zusammensetzung II zur Herstellung der Epoxy-Beschichtung**

| | Anteil |
|---|---|
| Epikote 828, Bindemittel der Fa. Hexion | 20 g |
| M1, 2, 3, 5-14, VG1-2, Benzylalkohol | 1 g |
| Vestamin IPD, Härter der Evonik Resource Efficiency GmbH | 4,54 g |

Das Bindemittel wurde in PE-Bechern vorgelegt, das erfindungsgemäße Modifizierungsmittel bzw. Benzylalkohol bzw. Vergleichbeispiele zudosiert und die Mischungen jeweils 1 min bei 1000 U/min in einem Speedmixer der Firma Hauschild eingearbeitet. Der Härter wurde zugewogen und die härtbare Zusammensetzungen 1 min bei 2000 U/min im Speedmixer gerührt. Daraus wurde eine Platte mit ca. 5 mm Schichtdicke gegossen. Die Carbamatbildung wurde nach 1, 2 und 7 Tagen begutachtet.

**Tabelle 6:**

| **Carbamatbildung auf einer Beschichtung enthaltend erfindungsgemäße Modifizierungsmitteln im Vergleich zu Benzylalkohol** | | | | |
|---|---|---|---|---|
| **Zusammensetzung II mit** | **Aufbau** | **nach 1 d** | **nach 2 d** | **nach 7 d** |
| Benzylalkohol | Benzylalkohol | 5 | 5 | 5 |
| M1 | Benzylalkohol + 3 EO | 5 | 5 | 5 |
| M1Ac | Benzylalkohol + 3 EO, acetyliert | 3 | 3 | 3 |
| M1Me | Benzylalkohol + 3 EO, methyliert | 2 | 2 | 3 |
| M2 | Benzylalkohol + 6 EO | 1 | 4 | 5 |
| M3 | Benzylalkohol + 3 EO/3 PO | 2 | 4 | 4 |
| M5 | Benzylalkohol + 2 EO/2 PO | 1 | 2 | 5 |
| M5Ac | Benzylalkohol + 2 EO/2 PO, acetyliert | 2 | 3 | 3 |
| M5Me | Benzylalkohol + 2 EO/2 PO, methyliert | 2 | 2 | 3 |
| M6 | Benzylalkohol + 1 EO/5 PO | 3 | 4 | 5 |
| M7 | Benzylalkohol + 2 EO/4 PO | 3 | 3 | 5 |
| M8 | Benzylalkohol + 1 EO/3 PO | 2 | 4 | 4 |
| M9 | Benzylalkohol + 1 PO + 3 EO | 5 | 5 | 5 |
| M10 | Benzylalkohol + 3 EO + 1 PO | 5 | 5 | 5 |
| M11 | Benzylalkohol + 1 EO/3 PO/2 BO | 3 | 3 | 3 |
| M12 | Benzylalkohol + 1 EO/3 PO/2 SO | 3 | 2 | 3 |
| M13 | Benzylalkohol + 2 EO/1 PO | 5 | 5 | 5 |
| M14 | Benzylalkohol + 1 EO/2 PO | 3 | 4 | 4 |
| VG1 | Benzylalkohol + 3 PO | 2 | 2 | 3 |
| VG2 | Benzylalkohol + 6 PO | 1 | 2 | 3 |

Beschichtungen mit erfindungsgemäßen Modifizierungsmitteln mit Ethoxy-Fragmenten zeigen vergleichbare Feuchtigkeitsempfindlichkeit wie die Beschichtung mit Benzylalkohol. Vergleichsbeispiele nur mit Propoxy-Fragmenten sind deutlich unterlegen.

## Patentansprüche

1. Modifizierungsmittel enthaltend Benzylalkohol-basierte Alkoxylate für härtbare Zusammensetzungen mit mindestens einem Epoxidharz und einem Härter, **dadurch gekennzeichnet, dass** das Benzylalkohol-basierte Alkoxylat durch Umsetzung von Benzylalkohol mit Alkylenoxiden erhalten wird, wobei das Benzylalkohol-basierte Alkoxylat mindestens ein Ethoxy-Fragment aufweist.

2. Modifizierungsmittel nach Anspruch 1, **dadurch gekennzeichnet, dass** es sich bei dem Benzylalkohol-basierten Alkoxylat um Benzylalkohol-basierte Ethoxylate oder Benzylalkohol-basierte Mischalkoxylate handelt, gemäß der Formel (I) 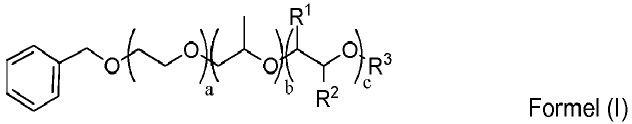 wobei
a = Alkoxy-Fragment (a) = 1 bis 10, bevorzugt 2 bis 7, besonders bevorzugt 3 bis 6,
b = Alkoxy-Fragment (b) = 0 bis 10, bevorzugt 1 bis 7, besonders bevorzugt 1 bis 5,
c = Alkoxy-Fragment (c) = 0 bis 10, bevorzugt 0 bis 6, besonders bevorzugt 0 bis 3,
R¹, R² = unabhängig voneinander Wasserstoff, eine Alkylgruppe mit 2 bis 20 Kohlenstoffatomen, eine Aryl- oder Alkarylgruppe, bevorzugt Ethyl-, Octyl-, Decyl-, Phenylgruppe mit der Maßgabe, dass R¹ ungleich H, wenn R² gleich Methyl, und dass nicht beide Reste R¹ und R² gleichzeitig H sein dürfen, R³ = unabhängig voneinander ein Wasserstoffradikal, eine Acetyl-, eine Phosphorester- oder eine Alkylgruppe mit 1 bis 20 Kohlenstoffatomen, die gegebenenfalls auch weiter substituiert sein kann, bevorzugt eine Acetyl-, Methyl-, Phosphorestergruppe oder ein Wasserstoffradikal, besonders bevorzugt ein Wasserstoffradikal.

3. Modifizierungsmittel nach Anspruch 2, **dadurch gekennzeichnet, dass** es sich bei den Alkylenoxiden vorzugsweise um Ethylenoxid (EO), Propylenoxid (PO), 1,2-Epoxy-2-methylpropan (Isobutylenoxid), Epichlorhydrin, 2,3- Epoxy-1-propanol, 1,2-Epoxybutan (Butylenoxid, BO), 2,3-Epoxybutan, 2,3-Dimethyl-2,3-epoxybutan, 1,2-Epoxypentan, 1,2-Epoxy-3-methylpentan, 1,2-Epoxyhexan, 1,2-Epoxycyclohexan, 1,2-Epoxyheptan, 1,2-Epoxyoctan, 1,2-Epoxynonan, 1,2-Epoxydecan, 1,2-Epoxyundecan, 1,2-Epoxydodecan, Styroloxid (SO), 1,2-Epoxycyclopentan, 1,2-Epoxycyclohexan, Vinylcyclohexenoxid, (2,3-Epoxypropyl)benzol, Vinyloxiran, 3-Phenoxy-1,2-epoxypropan, 2,3-Epoxymethylether, 2,3-Epoxyethylether, 2,3- Epoxylisopropylether, (3,4-Epoxybutyl)stearat, 4,5-Epoxypentylacetat, 2,3-Epoxylpropanmethacrylat, 2,3-Epoxylpropanacrylat, Gylcidylbutyrat, Methylglycidat, Ethyl-2,3-epoxybutanoat, 4-(Trimethylsilyl)butan-1,2-epoxid, 4-(Triethylsilyl)butan-1,2-epoxid, 3-(Perfluoromethyl)-1,2-epoxypropan, 3- (Perfluoroethyl)-1,2-epoxypropan, 3-(Perfluorobutyl)-1,2-epoxypropan, 3- (Perfluorohexyl)-1,2-epoxypropan, 4-(2,3-Epoxypropyl)morpholin, 1- (Oxiran-2-ylmethyl)pyrrolidin-2-on handelt.

4. Modifizierungsmittel nach Anspruch 3, **dadurch gekennzeichnet, dass** die Alkylenoxide einzeln oder in beliebigen Mischungen zur Alkoxylierung des Benzylalkohols eingesetzt werden.

5. Modifizierungsmittel nach Anspruch 4, **dadurch gekennzeichnet, dass** das Benzylalkohol-basierte Mischalkoxylat durch Umsetzung mit Ethylenoxid (EO) und Propylenoxid (PO) in einem molaren Verhältnis von 1 : 10 bis 10 : 0, bevorzugt von 3 : 1 bis 1 : 0 hergestellt wird.

6. Modifizierungsmittel nach Anspruch 5, **dadurch gekennzeichnet, dass** die Alkoxy-Fragmente (a) und (b) statistisch oder blockartig verteilt an den Benzylalkohol angelagert werden können.

7. Modifizierungsmittel nach Anspruch 2, **dadurch gekennzeichnet, dass** der Alkoxylierungsgrad 3 bis 10, vorzugsweise 4 bis 8 beträgt.

8. Modifizierungsmittel nach Anspruch 2, **dadurch gekennzeichnet, dass** das Benzylalkohol-basierte Alkoxylat eine terminale Hydroxygruppe aufweist.

9. Modifizierungsmittel nach einem der vorstehenden Ansprüche, **dadurch gekennzeichnet, dass** die terminale Hydroxygruppe des Benzylalkohol-basierten Alkoxylats acetyliert, methyliert oder phosphoryliert wird.

10. Modifizierungsmittel nach Anspruch 2, **dadurch gekennzeichnet, dass** die Polydispersität (Mw/Mn) der Benzylalkohol-basierten Mischalkoxylate gemäß Formel (I) < 2,5, bevorzugt < 2,0 und besonders bevorzugt von >1,05 bis <1,5, bestimmt mittels GPC, beträgt.

11. Verwendung von Benzylalkohol-basierten Alkoxylaten, wobei das Benzylalkohol-basierte Alkoxylat mindestens ein Ethoxy-Fragment aufweist, als Modifizierungsmittel für härtbare Zusammensetzungen enthaltend mindestens ein Epoxidharz, mindestens einen Härter, vorzugsweise einen aminischen Härter oder einen sauren Härter, und optional weitere Hilfskomponenten.

12. Verwendung von Benzylalkohol-basierten Alkoxylaten nach Anspruch 11 gemäß Formel (I).

13. Härtbare Zusammensetzung enthaltend
(1) mindestens ein Epoxidharz,
(2) mindestens einen Härter, vorzugsweise (2a) aminischer Natur oder (2b) saurer Natur,
(3) mindestens ein Modifizierungsmittel nach einem der Ansprüche 1 - 10,
(4) gegebenenfalls weitere Hilfskomponenten.

14. Härtbare Zusammensetzung nach Anspruch 13, **dadurch gekennzeichnet, dass** Bisphenol A-diglycidylether-basierte Epoxidharze oder Bisphenol F-diglycidylether-basierte Epoxidharze eingesetzt werden.

15. Härtbare Zusammensetzung nach Anspruch 14, **dadurch gekennzeichnet, dass** aliphatische, cycloaliphatische, araliphatische oder aromatische Amine oder Polyamine als aminische Härter (2a) eingesetzt werden.

16. Härtbare Zusammensetzung nach Anspruch 15, **dadurch gekennzeichnet, dass** Säuren und Säureanhydride als saure Härter (2b) eingesetzt werden.

17. Verwendung von härtbaren Zusammensetzungen nach einem der Ansprüche 13 - 16 für Fußbodenbeschichtungen, Lacke, Polymerbeton, Reparatursysteme, Ankermassen, Klebstoffe, Vergussmassen und Imprägnierungen, Faserverbundwerkstoffe.
